(19) Europäisches Patentamt
European Patent Office
Office européen des brevets

(11) Numéro de publication : **0 060 171**
**B1**

(12) **FASCICULE DE BREVET EUROPÉEN**

(45) Date de publication du fascicule du brevet : **15.05.85**

(21) Numéro de dépôt : **82400303.2**

(22) Date de dépôt : **22.02.82**

(51) Int. Cl.[4] : **C 07 C 85/04**, C 07 C 87/62, C 07 D207/32, C 07 D209/48, C 07 D235/06, C 07 D279/18

(54) **Procédé de N-alkylation de composés organiques azotés.**

(30) Priorité : **11.03.81 FR 8104823**

(43) Date de publication de la demande : **15.09.82 Bulletin 82/37**

(45) Mention de la délivrance du brevet : **15.05.85 Bulletin 85/20**

(84) Etats contractants désignés : **AT BE CH DE FR GB IT LI LU NL SE**

(56) Documents cités :
**FR-A- 2 459 792**
**US-A- 4 155 936**
**CHEMICAL ABSTRACTS, vol. 89, no. 3, 17 juillet 1978 réf.: 24076t, page 630 COLUMBUS, OHIO (US) N.C. WANG et al.: Pyrrole chemistry XVII. Alkylation of the pyrrolyl ambident anion"**
**CHEMICAL ABSTRACTS, vol. 94, no. 15, 13 avril 1981 réf.: 121221x, page 669 COLUMBUS, OHIO (US) E. SANTANIELLO et al.: "Preparation of N-alkylphthalimides assisted by solid-liquid phase-transfer systems"**
**JOURNAL OF ORGANIC CHEMISTRY, vol. 45, no. 16, 1er août 1980 American Chemical Society W.C. GUIDA et al.: "Phase-transfer alkylation of heterocycles in the presence of 18-Crown-6 and potassium tert-butoxide" pages 3172-3176**

(73) Titulaire : **RHONE-POULENC SPECIALITES CHIMIQUES**
**"Les Miroirs" 18, Avenue d'Alsace**
**F-92400 Courbevoie (FR)**

(72) Inventeur : **Soula, Gérard**
**33, rue Nungesser**
**F-69330 Meyzieu (FR)**
Inventeur : **Balme, Maurice**
**23, boulevard du 11 Novembre**
**F-69110 Sainte-Foy-lès-Lyon (FR)**

(74) Mandataire : **Cazes, Jean-Marie et al**
**RHONE-POULENC RECHERCHES Service Brevets Chimie et Polymères 25, quai Paul Doumer**
**F-92408 Courbevoie Cedex (FR)**

**EP 0 060 171 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention a pour objet un procédé de N-alkylation de composés organiques azotés ; elle concerne plus particulièrement la réaction d'un composé organique comportant un atome d'azote auquel est lié un hydrogène labile avec un agent alkylant en présence d'une base.

On connaît dans l'art antérieur un certain nombre de procédés de ce type.

C'est ainsi que D. H JONES décrit dans J. Chem. Soc. (c) 1971.132 la réaction de phénothiazines substituées avec des halogénoalcanes substitués comme la N-N diméthyl amino-3 chloropropane dans le diméthylformamide en présence d'hydrure de sodium. L'inconvénient principal de ce type de procédé réside dans l'utilisation d'un solvant polaire aprotique dont l'homme de l'art connaît bien les difficultés de mise en œuvre à l'échelle industrielle. De plus, l'hydrure de sodium est un produit cher et dangereux.

Cette même réaction a été mise en œuvre en catalyse par transfert de phase liquide-liquide. La technique décrite dans Synthesis (1977, p. 342 J. MASSE) fait intervenir un système biphasique eau-benzène en présence d'un ammonium quaternaire pour alkyler la chloro-2 phénothiazine. Les résultats obtenus avec les agents alkylants chlorés ($C_6H_5—CH_2—Cl$, $(H_3C)_2N—(CH_2)_3—Cl$) sont négatifs (rendements de 0 à 20 %). Les résultats obtenus avec les agents alkylants bromés ($H_2C = CH—CH_2—Br$, $C_2H_5Br$, $Cl(CH_2)_3Br$), composés plus réactifs, sont meilleurs (40-55 %) mais pas complètement satisfaisants au plan industriel.

Cette même technique de transfert de phase liquide-liquide a aussi été mise en œuvre par H. J.-M. DOU et J. METZGER qui décrivent la N-alkylation de composés hétérocycliques comme le pyrazole et l'imidazole avec divers agents alkylants. Seuls les agents alkylants réactifs comme le bromo-1 butane, le bromo-1 phénoxy-3 propane et le chlorure de benzyle donnent de bons rendements (70-80 %). Les agents alkylants non réactifs comme le chloro-1 octane, le bromure de butyle tertiaire, le bromure de dodécyle donnent des rendements négligeables (0 à 5 %).

On connaît encore l'article de Wayne C. GUIDA et David J. MATHRE dans J. Org. Chem. 1980, 45-3172 qui décrit la N-alkylation de composés hétérocycliques azotés comportant un atome d'hydrogène labile lié à l'atome d'azote. Le procédé décrit est un procédé par transfert de phase selon lequel on fait réagir le substrat et l'agent alkylant dans de l'éther diéthylique en présence d'une base et d'un éther couronne (« 18-Crown-6 »). Cet article ne cite que des agents alkylants réactifs comme l'iodure de méthyle, le bromure de méthyle, l'iodure d'éthyle, le bromure d'allyle. Il est à noter que ce procédé est d'une utilisation industrielle délicate dans la mesure où les éthers-couronnes sont des composés très onéreux ce qui, bien entendu, limite leur intérêt dans les procédés où l'aspect économique est important.

On voit donc que subsiste dans l'art antérieur le besoin d'un procédé de N-alkylation de composés azotés qui, d'une part ne met pas en œuvre de solvants et de réactifs de manipulation industrielle délicate et, d'autre part, permet l'utilisation d'agents alkylants peu réactifs, ces derniers étant généralement des composés plus facilement disponibles.

Le procédé de l'invention atteint cet objectif.

La présente invention a donc pour objet un procédé de N-alkylation de composés organiques azotés, caractérisé en ce que l'on fait réagir le composé organique azoté comportant, lié à l'atome d'azote, un hydrogène labile avec un agent alkylant en présence d'une part, d'une base minérale et, d'autre part, d'un agent séquestrant de formule générale :

$$N[—CHR_1—CHR_2—O—(CHR_3—CHR_4—O)_n—R_5]_3 \qquad (I)$$

dans laquelle n est un nombre entier supérieur ou égal à 0 et inférieur ou égal à 10 ($0 \leq n \leq 10$), $R_1$, $R_2$, $R_3$, $R_4$ identiques ou différents représentent un atome d'hydrogène ou un radical alkyle ayant de 1 à 4 atomes de carbone et $R_5$ représente un radical alkyle ou cycloalkyle ayant de 1 à 12 atomes de carbone, un radical phényle ou un radical de formule $—C_mH_{2m}—\Phi$, ou $C_mH_{2m+1}—\Phi—$, m étant compris entre 1 et 12.

Ce sont des considérations essentiellement économiques qui fixent la valeur supérieure de n à 10. En effet, les composés de formule I pour lesquels n serait supérieur à 10, conviendrait pour la mise en œuvre du procédé selon l'invention, mais leur synthèse se heurterait à des difficultés qui se répercuteraient sur l'économie du procédé.

Les composés organiques azotés concernés par la présente invention peuvent être, soit des composés hétérocycliques azotés, soit des anilines substituées de formule générale :

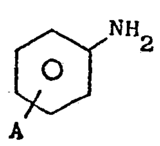

(II)

où A situé en ortho et/ou para représente au moins un groupement électroattracteur permettant à l'atome d'hydrogène lié à l'atome d'azote d'avoir un caractère suffisamment acide.

On peut citer comme exemples non limitatifs de tels composés : la paranitroaniline, l'orthonitroani-

line, la paracyanoaniline, l'orthocyanoaniline, l'orthotrifluorométhylaniline et la paratrifluorométhylaniline, la dinitro-2,4 aniline.

On peut citer comme exemples non limitatifs de composés hétérocycliques azotés, le pyrole, l'indole, le pyrazole, l'imidazole, le benzimidazole, le benzotriazole, le carbazole, la phénothiazine, le phtalimide et leurs dérivés.

L'agent alkylant pouvant être mis en œuvre dans le procédé de l'invention peut être représenté par la formule générale :

$$R_6—X \qquad (III)$$

où

— $R_6$ représente un radical choisi parmi le groupe comprenant :
les radicaux alkyle ayant de 1 à 12 atomes de carbone éventuellement substitués,
le radical benzyle éventuellement substitué,
les radicaux allyle éventuellement substitués,

— et X représente un élément choisi parmi le groupe comprenant Cl, Br, I, les radicaux alkylsulfonate et arylsulfonate.

Les composés présentant le plus d'intérêt sont les composés où X est un chlore.

On peut citer comme exemples d'agents alkylants : les chloroalcanes comme le chlorure de méthyle, d'éthyle, d'isopropyle, le chlorure d'hexyle, le chlorooctane, le N-N diméthyl amino-3 chloropropane, le dichloro-1,2 éthane, le chlorure de benzyle, le parachlorure de benzyle, le chlorure d'allyle, le chlorure de métallyle. On peut également citer les composés bromés correspondants ; leur utilisation est d'une façon générale moins intéressante pour les raisons développées ci-dessus.

La base minérale mise en œuvre dans le procédé de l'invention est choisie de préférence parmi le groupe comprenant les hydroxydes, les hydrogénocarbonates et les carbonates des métaux alcalins et alcalino-terreux. On peut citer plus particulièrement comme exemples de telles bases : la soude, la lithine, la potasse, l'hydrogénocarbonate de sodium, le carbonate de sodium, le carbonate de potassium et la chaux.

Selon un mode de réalisation préférentiel du procédé de l'invention, on utilise au moins un agent séquestrant de formule (I) dans laquelle $R_1$, $R_2$, $R_3$ et $R_4$ représentent un atome d'hydrogène ou un radical méthyle, $R_5$ et n ayant la signification précédente.

Parmi ces derniers, on préfère encore plus particulièrement mettre en œuvre les agents séquestrants pour lesquels n est supérieur ou égal à 0 et inférieur ou égal à 6, et pour lesquels $R_5$ représente un radical alkyle ayant de 1 à 4 atomes de carbone.

On peut citer :

— la tris(oxa-3 butyl)amine de formule :

$$N—(CH_2—CH_2—O—CH_3)_3$$

— la tris(oxa-3 heptyl)amine de formule :

$$N—(CH_2—CH_2—O—C_4H_9)_3$$

— la tris(dioxa-3,6 heptyl)amine de formule :

$$N—(CH_2—CH_2—O—CH_2—CH_2—O—CH_3)_3$$

— la tris(trioxa-3,6,9 décyl)amine de formule :

$$N—(CH_2—CH_2—O—CH_2—CH_2—O—CH_2—CH_2—O—CH_3)_3$$

— la tris(dioxa-3,6 octyl)amine de formule :

$$N—(CH_2—CH_2—O—CH_2—CH_2—O—C_2H_5)_3$$

— la tris(trioxa-3,6,9 undécyl)amine de formule :

$$N—(CH_2—CH_2—O—CH_2—CH_2—O—CH_2—CH_2—O—C_2H_5)_3$$

— la tris(dioxa-3,6 nonyl)amine de formule :

$$N—(CH_2—CH_2—O—CH_2—CH_2—O—C_3H_7)_3$$

— la tris(trioxa-3,6,9 dodécyl)amine de formule :

$$N—(CH_2—CH_2—O—CH_2—CH_2—O—CH_2—CH_2—O—C_3H_7)_3$$

— la tris(dioxa-3,6 décyl)amine de formule :

$$N—(CH_2—CH_2—O—CH_2—CH_2—O—C_4H_9)_3$$

— la tris(trioxa-3,6,9 tridécyl)amine de formule :

$$N—(CH_2—CH_2—O—CH_2—CH_2—O—CH_2—CH_2—O—C_4H_9)_3$$

— la tris(oxa-3 butyl)amine de formule :

$$N—(CH_2—CH_2—O—CH_3)_3$$

— la tris(tétraoxa-3,6,9,12 tridécyl)amine de formule :

$$N—[CH_2—CH_2—O—(CH_2—CH_2—O—)_3—CH_3]_3$$

On peut encore citer :

— la tris(dioxa-3,6 méthyl-4 heptyl)amine de formule :

$$N—(CH_2—CH_2—O—CHCH_3—CH_2—O—CH_3)_3$$

— la tris(dioxa-3,6 diméthyl-2,4 heptyl)amine de formule :

$$N—(CH_2—CHCH_3—O—CHCH_3—CH_2—O—CH_3)_3$$

Le procédé selon l'invention peut être mis en œuvre en présence ou non d'un tiers solvant. Quand on en utilise un, on le choisit de préférence parmi les solvants aprotiques apolaires ou aprotiques peu polaires comme par exemple le benzène, le toluène, le chlorobenzène, le dichlorobenzène, le dichlorométhane et l'acétonitrile.

On peut ne pas utiliser de tiers solvant ; c'est alors l'agent alkylant qui joue le rôle de solvant.

On préférera utiliser un solvant lorsque l'agent alkylant mis en œuvre est un produit difficilement accessible ou cher ; il en sera de même s'il est trop réactif (dans ce cas il est préférable de le diluer dans un tiers solvant).

On utilise, de préférence, selon le procédé de l'invention, le composé organique azoté et l'agent alkylant en quantités telles que le rapport molaire du premier au second est compris entre 0,1 et 5. Les hautes valeurs de ce rapport correspondent au cas où l'agent alkylant est utilisé comme solvant.

Le rapport molaire de l'agent séquestrant au composé organique azoté est, de préférence, compris entre 0,01 et 0,1. Encore plus préférentiellement, ce rapport est compris entre 0,02 et 0,08.

Pour une bonne mise en œuvre de l'invention, la base minérale est utilisée en quantité telle que le rapport molaire de la base minérale au composé organique azoté est de préférence compris entre 1 et 5 ; encore plus préférentiellement ce rapport est compris entre 1 et 2.

Le procédé selon l'invention est mis en œuvre, de préférence, à une température comprise entre 0 °C et 200 °C, à la pression atmosphérique. Des pressions supérieures ou inférieures à la pression atmosphérique ne sont pas exclues du domaine de l'invention.

Les composés obtenus selon le procédé de l'invention sont les composés organiques azotés de départ comportant en lieu et place de l'atome d'hydrogène labile, fixé sur l'atome d'azote, le radical $R_6$ de l'agent alkylant. Ils sont utilisés comme intermédiaires dans la synthèse de nombreux composés organiques.

Les agents séquestrants mis en œuvre dans le procédé de l'invention peuvent être préparés selon le mode opératoire décrit dans la demande de brevet français 7 905 438 publiée sous le numéro 2 450 120.

L'invention va être maintenant plus complètement illustrée à l'aide des exemples qui vont suivre. Ceux-ci ne peuvent être considérés comme une limitation quelconque de l'invention.

Exemple 1

N-alkylation du phtalimide

Dans un réacteur de 150 ml équipé d'un agitateur magnétique, d'un condenseur et d'un thermomètre, on introduit successivement : 20 $cm^3$ d'acétonitrile 0,025 mole de phtalimide (3,67 g), 0,025 mole de carbonate de potassium (3,45 g) 0,025 mole de bromodécane (5,5 g) et 0,001 mole de tris(dioxa-3,6 heptyl)amine (0,32 g).

Le mélange est chauffé à reflux sous agitation pendant 6 heures puis refroidi et analysé par chromatographie en phase gazeuse.

Au moyen d'étalons, on détermine le rendement de la réaction ; il est égal à 93 %.

Essai comparatif

En l'absence de la tris(dioxa-3,6 heptyl)amine, toutes choses étant égales par ailleurs, le rendement est de 42 %.

Exemple 2

N-alkylation du phtalimide

On opère comme dans l'exemple 1 mais en remplaçant l'acétonitrile par le chlorobenzène. Après 8 heures à reflux, le rendement est de 70 %.

Essai comparatif

Sans catalyseur, le rendement est de 0 %.

Exemple 3

N-alkylation du benzimidazole

Dans un réacteur de 150 ml équipé d'un agitateur magnétique, d'un condenseur et d'un thermomètre, on introduit successivement 20 $cm^3$ de toluène 0,02 mole de chlorooctane (2,97 g), 0,02 mole de potasse, 0,02 mole de benzimidazole (2,36 g) et 0,002 mole de tris(dioxa-3,6 heptyl)amine (0,64 g). Le mélange est chauffé à reflux sous agitation pendant 5 heures puis refroidi et analysé par chromatographie en phase vapeur. Au moyen d'étalons, on détermine le rendement de la réaction qui est égal à 76 %.

Essai comparatif

En l'absence de la tris(dioxa-3,6 heptyl)amine, le rendement est de 10 %.

Exemple 4

N-alkylation du pyrole

Dans le réacteur défini à l'exemple 1, on introduit successivement 20 $cm^3$ de toluène, 0,01 mole de pyrole (0,67 g) 0,01 mole de potasse (0,65 g), 0,01 mole de bromohexane (1,65 g) et 0,16 g de tris(dioxa-3,6 octyl)amine (0,000 5 mole). Après 4 heures de chauffage à reflux, le rendement de la réaction déterminée par analyse chromatographique en phase gazeuse est de 95 %.

Essai comparatif

Sans catalyseur, dans les mêmes conditions, le rendement est de 0 %.

Exemple 5

N-alkylation de la para-nitroaniline

Dans un réacteur de 100 ml équipé d'un agitateur magnétique et d'un condenseur à reflux, on introduit successivement : 20 $cm^3$ de toluène anhydre, 1,31 g de potasse (0,02 mole), 3,3 g de bromohexane (0,02 mole), 2,76 g de para-nitroaniline (0,02 mole) et 0,32 g de tris(dioxa-3,6 heptyl)amine ($10^3$ mole). Le mélange est porté à reflux pendant une heure puis refroidi et lavé avec 20 $cm^3$ d'eau ; la phase organique est récupérée, séchée et le toluène est distillé. Par analyses infra-rouge et spectrométrie de masse, la N-hexyl para nitro aniline est identifiée. Le rendement de la réaction est de 86 %.

Dans les mêmes conditions, en l'absence de la tris(dioxa-3,6 heptyl)amine, la réaction n'a pas lieu.

Exemple 6

N-alkylation de la chloro-2 phénothiazine

Dans un réacteur de 125 ml sont chargés : 4,07 g de chloro-2 phénothiazine (soit 0,017 43 mole), 0,785 g de soude en pastille de pureté 97,7 % (soit 0,019 2 mole finement broyée : excès 10 %), 0,630 g de tris(dioxa-3,6 octyl)amine (0,001 75 mole) et 65 ml de toluène.

Le réacteur est immergé dans un banc thermorégularisé à 100 °C. Quand la masse est à reflux, on introduit en 2 h : 2,60 g de diméthylamino-3 méthyl-2 chloro-1 propane, en solution à 578 g/l dans le toluène. Trois heures après la fin de coulée du diméthylamino-3 méthyl-2 chloro-1 propane, la masse réactionnelle est refroidie, coulée dans l'eau. La phase organique est décantée, lavée à l'eau.

L'analyse par chromatographie en phase gazeuse montre que l'on a formé 0,010 3 mole de chloro-2 alimémazine, de formule

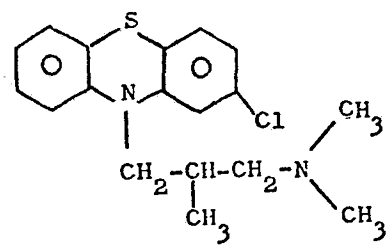

Le taux de transformation est de 65 % et le rendement est de 59 %.

Essai comparatif

On opère dans les mêmes conditions que ci-dessus mais sans tris(dioxa-3,6 octyl)amine et avec les réactifs suivants : 4,56 g de chloro-2 phénothiazine, 0,888 g de soude en poudre de pureté 97,8 % (soit 0,021 7 mole), 5.1 ml de diméthylamino-3 méthyl-2 chloro-1 propane en solution toluénique à 578 g/litre.

On introduit à reflux en 2 h 10 la solution toluénique de diméthylamino-3 méthyl-2 chloro-1 propane et on laisse ensuite 3 h de réaction à reflux.

Le taux de transformation est de 13 %.

Exemple 7

N-alkylation de la chloro-2 phénothiazine

On opère comme dans l'exemple 6 en remplaçant la soude par la même quantité molaire de potasse (de pureté 86,8 %).

Le taux de transformation est de 98 % et le rendement de 94 %.

Essai comparatif

On opère comme dans l'essai comparatif de l'exemple 6 mais en remplaçant la soude par la même quantité molaire de potasse (de pureté = 86,8 %).

Le taux de transformation est de 89 % et le rendement est de 86 %.

**Revendications**

1. Procédé de N-alkylation de composés organiques azotés, caractérisé en ce que l'on fait réagir le composé organique azoté comportant, lié à l'atome d'azote, un hydrogène labile avec un agent alkylant en présence d'une part, d'une base minérale et, d'autre part, d'un agent séquestrant de formule générale :

$$N[-CHR_1-CHR_2-O-(CHR_3-CHR_4-O)_n-R_5]_3 \quad (I)$$

dans laquelle n est un nombre entier supérieur ou égal à 0 et inférieur ou égal à 10 ($0 \leq n \leq 10$), $R_1$, $R_2$, $R_3$, $R_4$ identiques ou différents représentent un atome d'hydrogène ou un radical alkyle ayant de 1 à 4 atomes de carbone et $R_5$ représente un radical alkyle ou cycloalkyle ayant de 1 à 12 atomes de carbone, un radical phényle ou un radical de formule $-C_mH_{2m}-\Phi$, ou $C_mH_{2m+1}-\Phi-$, m étant compris entre 1 et 12.

2. Procédé selon la revendication 1, caractérisé en ce que l'on utilise au moins un agent séquestrant de formule (I) dans laquelle $R_1$, $R_2$, $R_3$ et $R_4$ représentent un atome d'hydrogène ou un radical méthyle.

3. Procédé selon l'une quelconque des revendications précédentes caractérisé en ce que l'on utilise au moins un agent séquestrant de formule (I) dans laquelle n est supérieur ou égal à 0 et inférieur ou égal à 6 et $R_5$ représente un radical alkyle ayant de 1 à 4 atomes de carbonne.

4. Procédé selon l'une quelconque des revendications précédentes caractérisé en ce que le composé organique azoté est choisi parmi le groupe comprenant les composés hétérocycliques azotés et les anilines substituées :

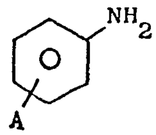

(II)

où A situé en ortho ou para représente un groupe électroattracteur.

5. Procédé selon la revendication 4, caractérisé en ce que les composés hétérocycliques azotés sont choisis parmi le groupe comprenant le pyrole, l'indole, le pyrazole, l'imidazole, le benzimidazole, le carbazole, la phénotiazine et le phtalimide.

6. Procédé selon la revendication 4, caractérisé en ce que les composés de formule (II) sont choisis parmi le groupe comprenant la paranitroaniline, l'orthonitroaniline, la paracyanoaniline, l'orthocyanoaniline, la paratrifluorométhylaniline et l'orthotrifluorométhylaniline.

7. Procédé selon l'une quelconque des revendications précédentes caractérisé en ce que l'agent alkylant a pour formule générale :

$$R_6—X \qquad (III)$$

dans laquelle $R_6$ représente un radical choisi parmi le groupe comprenant les radicaux alkyle ayant de 1 à 12 atomes de carbone éventuellement substitués et les radicaux allyle éventuellement substitués et X représente un radical choisi parmi le groupe comprenant Cl, Br, I et les radicaux alkylsulfonates et arylsulfonates.

8. Procédé selon la revendication 7, caractérisé en ce que X représente Cl.

9. Procédé selon l'une quelconque des revendications précédentes, caractérisé en ce que la base minérale est choisie parmi le groupe comprenant les hydroxydes, les hydrogénocarbonates et les carbonates de métaux alcalins et alcalino-terreux.

10. Procédé selon l'une quelconque des revendications précédentes, caractérisé en ce que l'on opère en présence d'un solvant.

11. Procédé selon la revendication 10, caractérisé en ce que le solvant est un solvant aprotique apolaire ou peu polaire.

12. Procédé selon l'une quelconque des revendications précédentes, caractérisé en ce que l'on met en œuvre le composé organique azoté et l'agent alkylant en quantités telles que le rapport molaire du premier au second est compris entre 0,1 et 5.

13. Procédé selon l'une quelconque des revendications précédentes caractérisé en ce que le rapport molaire de l'agent séquestrant au composé organique azoté est compris entre 0,01 et 0,1.

14. Procédé selon l'une quelconque des revendications précédentes, caractérisé en ce que la base minérale est utilisée en quantités telles que le rapport molaire de la base minérale au composé organique azoté est compris entre 1 et 5.

## Claims

1. Process for N-alkylation of nitrogenous organic compounds, characterised in that the nitrogenous organic compound, which incorporates a labile hydrogen attached to the nitrogen atom, is reacted with an alkylating agent in the presence of, on the one hand an inorganic base, and on the other hand a sequestering agent of general formula :

$$N[—CHR_1—CHE_2—O—(CHR_3—CHR_4—O)_n—R_5]_3 \qquad (I)$$

in which n is an integer greater than or equal to 0 and less than or equal to 10 ($0 \leqslant n \leqslant 10$), $R_1$, $R_2$, $R_3$ and $R_4$, which may be identical or different, denote a hydrogen atom or an alkyl radical having from 1 to 4 carbon atoms, and $R_5$ denotes an alkyl or cycloalkyl radical having from 1 to 12 carbon atoms, a phenyl radical or a radical of formula $—C_mH_{2m}—\Phi$ or $C_mH_{2m+1}—\Phi—$, m being between 1 and 12.

2. Process according to Claim 1, characterised in that at least one sequestering agent of formula (I) is used in which $R_1$, $R_2$, $R_3$ and $R_4$ denote a hydrogen atom or a methyl radical.

3. Process according to either one of the preceding claims, characterised in that at least one sequestering agent of formula (I) is used in which n is greater than or equal to 0 and less than or equal to 6, and $R_5$ denotes an alkyl radical having from 1 to 4 carbon atoms.

4. Process according to any one of the preceding claims, characterised in that the nitrogenous organic compound is chosen from the group comprising nitrogenous heterocyclic compounds and substituted anilines :

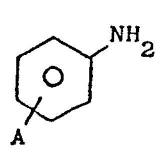

(II)

where A situated in the ortho- or para-position denotes an electron-attracting group.

5. Process according to Claim 4, characterised in that the nitrogenous heterocyclic compounds are chosen from the group comprising pyrrole, indole, pyrazole, imidazole, benzimidazole, carbazole, phenothiazine and phthalimide.

6. Process according to Claim 4, characterised in that the compounds of formula (II) are chosen from the group comprising para-nitroaniline, ortho-nitroaniline, para-cyanoaniline, ortho-cyanoaniline, para-(trifluoromethyl)aniline and ortho-(trifluoromethyl)aniline.

7. Process according to any one of the preceding claims, characterised in that the alkylating agent has the general formula :

$$R_6—X \quad (III)$$

in which $R_6$ denotes a radical chosen from the group comprising optionally substituted alkyl radicals having from 1 to 12 carbon atoms and optionally substituted allyl radicals, and X denotes a radical chosen from the group comprising Cl, Br, I and alkylsulphonate and arylsulphonate radicals.

8. Process according to Claim 7, characterised in that X denotes Cl.

9. Process according to any one of the preceding claims, characterised in that the inorganic base is chosen from the group comprising the hydroxides, hydrogen carbonates and carbonates of the alkali metals and alkaline earth metals.

10. Process according to any one of the preceding claims, characterised in that it is carried out in the presence of a solvent.

11. Process according to Claim 10, characterised in that the solvent is an aprotic, apolar or weakly polar solvent.

12. Process according to any one of the preceding claims, characterised in that the nitrogenous organic compound and the alkylating agent are used in quantities such that the mole ratio of the first to the second is between 0.1 and 5.

13. Process according to any one of the preceding claims, characterised in that the mole ratio of the sequestering agent to the nitrogenous organic compound is between 0.01 and 0.1

14. Process according to any one of the preceding claims, characterised in that the inorganic base is used in quantities such that the mole ratio of the inorganic base to the nitrogenous organic compound is between 1 and 5.

**Patentansprüche**

1. Verfahren zur N-Alkylierung von organischen Stickstoffverbindungen, dadurch gekennzeichnet, daß man die organische Stickstoffverbindung, die am Stickstoffatom ein labiles Wasserstoffatom trägt, mit einem Alkylierungsmittel umsetzt, in Gegenwart von einesteils einer anorganischen Base und anderenteils eines Sequestrierungsmittels der allgemeinen Formal :

$$N[—CHR_1—CHR_2—O—(CHR_3—CHR_4—O)_n—R_5]_3 \quad (I)$$

in der n ganzzahlige Werte von 1 bis 10 haben oder 0 sein kann ($0 \leq n \leq 10$), $R_1$, $R_2$, $R_3$, $R_4$, die gleich oder verschieden sein können, ein Wasserstoffatom oder ein Alkylradikal mit 1 bis 4 C-Atomen, und $R_5$ ein Alkyl- oder Cycloalkylradikal mit 1 bis 12 C-Atomen, ein Phenylradikal oder ein Radikal der Formel $—C_mH_{2m}—\Phi$ oder $C_mH_{2m+1}—\Phi—$, wobei m Werte von 1 bis 12 annimmt, bedeuten.

2. Verfahren nach Anspruch 1, dadurch gekennzeichnet, daß man wenigstens ein Sequestrierungsmittel der Formel I verwendet, in der $R_1$, $R_2$, $R_3$ und $R_4$ ein Wasserstoffatom oder ein Methylradikal bedeuten.

3. Verfahren nach einem der vorangehenden Ansprüche, dadurch gekennzeichnet, daß man wenigstens ein Sequestrierungsmittel der Formel (I) einsetzt, in der n Werte von 0 bis 6 besitzt und $R_5$ ein Alkylradikal mit 1 bis 4 Kohlenstoffatomen ist.

4. Verfahren nach einem der vorangehennden Ansprüche, dadurch gekennzeichnet, daß die organische Stickstoffverbindung ausgewählt wird aus der Gruppe, die die heterocyclischen Stickstoffverbindungen und die substituierten Aniline :

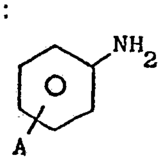

(II)

umfasst, in der der Substituent A in Ortho- oder Parastellung eine elektronenanziehende Gruppe ist.

5. Verfahren nach Anspruch 4, dadurch gekennzeichnet, daß die heterocyclischen Stickstoffverbindungen ausgewählt werden aus der Gruppe, die Pyrol, Indol, Pyrazol, Imidazol, Benzimidazol, Carbazol, Phenotiazin und Phtalimid umfasst.

6. Verfahren nach Anspruch 4, dadurch gekennzeichnet, daß die Verbindungen der Formel (II) ausgewählt werden aus der Gruppe, die Paranitroanilin, Orthonitroanilin, Paracyanoanilin, Orthocyanoanilin, Paratrifluormethylanilin und Orthotrifluormethylanilin umfasst.

7. Verfahren nach einem der vorangehenden Ansprüche, dadurch gekennzeichnet, daß das Alkylierungsmittel die allgemeine Formel :

$$R_6—X \qquad (III)$$

hat, in der $R_6$ ein aus der Gruppe, die die gegebenenfalls substituierten Alkylradikale mit 1 bis 12 Kohlenstoffatomen und die gegebenenfalls substituierten Allylradikale umfasst, ausgewähltes Radikal und X ein aus der Gruppe, die Cl, Br, I, die Alkylsulfonat- und Arylsulfonatgruppe umfasst, ausgewähltes Radikal ist.

8. Verfahren nach Anspruch 7, dadurch gekennzeichnet, daß X Cl ist.

9. Verfahren nach einem der vorangehenden Ansprüche, dadurch gekennzeichnet, daß die anorganische Base ausgewählt wird aus der Gruppe der Hydroxide, Hydrogencarbonate und Carbonate der Alkali- und Erdalkalimetalle.

10. Verfahren nach einem der vorangehenden Ansprüche, dadurch gekennzeichnet, daß man in Gegenwart eines Lösungsmittels arbeitet.

11. Verfahren nach Anspruch 10, dadurch gekennzeichnet, daß das Lösungsmittel ein unpolares oder wenig polares aprotisches Lösungsmittel ist.

12. Verfahren nach einem der vorangehenden Ansprüche, dadurch gekennzeichnet, daß man die organische Stickstoffverbindung und das Alkylierungsmittel in solchen Mengen einsetzt, daß das Molverhältnis zwischen Stickstoffverbindung und Alkylierungsmittel zwischen 0,1 und 5 liegt.

13. Verfahren nach einem der vorangehenden Ansprüche, dadurch gekennzeichnet, daß das Molverhältnis zwischen dem Sequestrierungsmittel und der organischen Stickstoffverbindung zwischen 0,01 und 0,1 liegt.

14. Verfahren nach einem der vorangehenden Ansprüche, dadurch gekennzeichnet, daß die anorganische Base in solchen Mengen eingesetzt wird, daß das Molverhältnis zwischen anorganischer Base und organischer Stickstoffverbindung zwischen 1 und 5 liegt.